(19) Europäisches Patentamt
European Patent Office
Office européen des brevets

(11) **EP 3 848 836 A1**

(12) **EUROPEAN PATENT APPLICATION**

(43) Date of publication:
**14.07.2021 Bulletin 2021/28**

(51) Int Cl.:
*G06F 21/57* (2013.01)     *G06F 21/62* (2013.01)
*G16H 50/20* (2018.01)     *G16H 50/70* (2018.01)
*G16H 10/20* (2018.01)     *G16H 10/60* (2018.01)
*G05B 13/00* (2006.01)     *G06N 3/08* (2006.01)
*A61B 5/02* (2006.01)

(21) Application number: **20150536.9**

(22) Date of filing: **07.01.2020**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
**KH MA MD TN**

(71) Applicant: **Robert Bosch GmbH**
**70442 Stuttgart (DE)**

(72) Inventors:
• **Zafar, Muhammad Bilal**
**71272 Renningen (DE)**
• **Rudolph, Maja Rita**
**72072 Tuebingen (DE)**
• **Schiegg, Martin**
**70825 Korntal-Muenchingen (DE)**
• **Gerwinn, Sebastian**
**71229 Leonberg (DE)**
• **Zimmer, Christoph**
**70825 Korntal (DE)**

(54) **PROCESSING A MODEL TRAINED BASED ON A LOSS FUNCTION**

(57)     The invention relates to a system (100) for processing a model. The model provides a model output given an input instance. The model has been trained on a training dataset by iteratively optimizing an objective function including losses according to a loss function for training instances of the training dataset. Upon receiving a removal request message identifying one or more undesired training instances of the training dataset, the model is made independent from the one or more undesired training instances. To this end, the one or more undesired training instances are removed from the training dataset to obtain a remainder dataset, and an adapted model is determined for the remainder dataset. The parameters of the adapted model are first initialized based on the set of parameters of the trained model, and then iteratively adapted by optimizing the objective function with respect to the remainder dataset.

EP 3 848 836 A1

Fig. 2

**Description**

**FIELD OF THE INVENTION**

The invention relates to a system for processing a model trained based on a loss function, and to a corresponding computer-implemented method. The invention further relates to a computer-readable medium comprising instructions to perform the method.

**BACKGROUND OF THE INVENTION**

Wearable devices such as smart watches, fitness trackers, and body-mounted sensors allow to measure and track various quantities of a user, for example, physiological quantities such as heart rate or blood pressure, or other kinds of physical quantities such as location, speed, rotational velocity, etcetera. Such measurements are then typically centrally collected and various services can be provided that make use of the measurements, for example, activity logging, sleep advise, etcetera. Many of these services apply machine learning models to the information collected from users, for example, to recognize patterns or to make predictions. A popular class of machine learning models is the class of so-called "Empirical Risk Minimization" (ERM)-type models, that are trained on a training dataset by iteratively optimizing an objective function including losses for respective training instances determined according to a, typically non-linear, loss function. Examples of such models include neural networks, or least square or logistic regression models. Apart from applying models to the information collected from users, services usually also use this information to further refine the machine learning models and thus improve their services. Also in many other settings, machine learning models are trained on personal information, for example, in medical image processing or facial recognition.

If a machine learning model is trained on a training dataset including personal information about a certain person, then this means that the machine learning model is dependent on that personal information in the sense that, if this personal information would not have been included in the dataset, the training would have led to a different model. In particular, the set of parameters of the trained model may be different. As a consequence, also for at least one input instance to which the trained model may be applied, the model trained using the personal information may provide a different model output from the model trained without the personal information. In some cases, due to these differences, it turns out to be possible to derive information about individuals in a dataset just from the trained model, a phenomenon known as "model inversion". More generally, since a trained model is effectively a function of the training dataset including their personal information, it would be desirable if a trained model could, if persons included in the training dataset so desired, be made substantially independent from training instances involving them. In fact, in many settings privacy regulations such as the General Data Protection Regulation (GDPR) of the European Union or the Health Insurance Portability and Accountability Act (HIPPA) of the United States may require up to various degrees to let a data subject control to what extent their personal information may be used, for example, to train machine learning models.

A known way of limiting the dependence of model outputs on any one particular training record is by making use of differentially private perturbation techniques. Differential privacy is a mathematical framework that specifies a maximal amount of deviation to model outputs due to the presence of absence of any single training record. In the setting of ERM-type models, in particular logistic regression models, "Privacy-preserving logistic regression" by K. Chaudhuri and C. Monteleoni, Advances in Neural Information Processing Systems, pages 289-296, 2009, proposes to use an existing trained model and add sufficient noise to its output to hide the effect of the presence of single records. Accordingly, due to the added noise, model outputs can be made to a large degree independent from a single training record.

**SUMMARY OF THE INVENTION**

Although noise may be added to model outputs of ERM-type models to make the model outputs more or less independent from a single training record, doing so provides only statistical guarantees that may be negated at least in part by collecting many model outputs and cancelling out their noise. Moreover, adding noise necessarily decreases the accuracy of the model outputs. Also, the approach of Chaudhuri and the framework of differential privacy more generally concerns the influence of single records on model outputs, and so may not be able to sufficiently limit the dependence of model outputs on multiple training records. Fundamentally, the more records the model would need to be made more independent of, the more noise would need to be added and thus the more accuracy would have to be sacrificed. Effectively, adding noise provides a trade-off in which making model outputs more independent from training records results in a lower accuracy of the model outputs that are obtained. Moreover, in Chaudhuri's approach, although adding noise reduces the privacy impact of model outputs, the existing model that is stored is still a function of the personal information and so may still allow model inversion and/or may still represent personal information. For example, in various situations, applying noise to model outputs may not be regarded as a sufficient measure to satisfy right-to-be-forgotten request arising due to the GDPR and similar other privacy regulations.

In accordance with a first aspect of the invention, a system for processing a model trained based on a loss function is proposed, as defined by claim 1. In accordance with another aspect of the invention, a corresponding computer-implemented method is proposed, as defined by claim 13. In accordance with an aspect of the invention, a computer-readable medium is provided as defined by claim 15.

In various embodiments, advantageously, a model trained based on a loss function may be made independent from one or more undesired training instances after the model has been trained and preferably also after the model has been deployed. For example, the model as deployed may initially depend on the one or more undesired training instances and, upon receiving a removal request message indicating the one or more undesired training instances, may be made independent of those training instances. By acting upon receiving a removal request message, interestingly, the model can be made independent from one or more *specific* training instances instead of having to make the model independent from any one training instance without knowing which. This way, for example, adding large amounts of noise to model outputs may be avoided.

Surprisingly, the inventors envisaged that, to this end, along with the trained model also the training dataset on which it has been trained may be kept. Accordingly, when the removal request comes in, a remainder dataset may be determined by removing the undesired training instances from the training dataset. Then, an adapted model for the remainder dataset may be determined by initializing the set of parameters of the adapted model based on the parameters of the trained model, and iteratively adapting the set of parameters of the adapted model by optimizing losses of instances of the remainder dataset with according to the loss function of the model. Making a model independent from undesired training instances may be referred to generally as "detraining" the model with respect to the undesired training instances.

By making the model independent from specific training instances and by doing so only upon receiving a removal request message for those specific instances, it may be enabled to still use the training instances while possible, e.g., while a data subject has not withdrawn consent. Moreover, by making the model independent from specific training instances, for example, it may be avoided to add generic noise that is large enough to hide any particular training instance. In fact, the parameters of the adapted model may be an optimum for the objective function used to train the model, e.g., both before and after dealing with the removal request message, the model may be able to provide model outputs with maximal accuracy given the records that the model output may be based on. For example, the adapted model may correspond to a model obtained by training on the remainder dataset.

By optimizing the objective function with respect to the remainder dataset, interestingly, an adapted model may be obtained that is independent from the one or more undesired training instances in the sense that the parameters of the adapted model may be obtained by optimizing an objective function that is independent from the one or more undesired training instances, e.g., the set of parameters may also be obtainable by training a model from scratch based on the remainder dataset. In that sense, the one or more undesired training instances may be regarded as being completely removed from the trained model. Accordingly, after dealing with the removal request, the undesired training records may be considered to be erased from the training dataset; from the trained model; and from the model outputs resulting from applying the trained model. It is noted that the adaptation of the trained model does not need to be performed by the same parties that apply the model to input instances, and in particular, not all parties that use the trained model need access to the training dataset. For example, a system may be configured to deal with removal request messages and, having determined an adapted model in response to one or more removal request messages, provide the adapted model to one or more other systems for applying the model to input instances. In such cases, although the system dealing with the removal request messages may need access to the training dataset, systems that obtain the adapted model and apply it may not need such access. Accordingly, the exposure of sensitive information may be limited, further improving security.

Interestingly, by initializing the parameters of the adapted model based on the parameters of the trained model, removal request messages may be dealt with efficiently. For example, a full re-training of the model based on the remainder dataset may be avoided. As the inventors realized, because the set of parameters of the trained model may be trained based on an objective function including losses for respective training instances, this set of parameters may be a relatively good initial estimate for an optimization with respect to the remainder dataset. For example, this set of parameters itself, or a value close to it, e.g., obtained by adding a relatively small amount of noise, may be used as an initial estimate for optimizing with respect to the remainder dataset. For example, the objective function may comprise a sum of losses of respective training instances. If relatively few training instances are removed, e.g., a single instance, at most ten, at most fifty, or at most 1% or at most 5% of the training dataset, then the objective function for the remainder dataset may mostly comprise the same losses as the objective function for the original dataset. Accordingly, an optimum of the objective function for the remainder dataset may be expected to be relatively close to an optimum of the objective function for the original training dataset. As a consequence, relatively few iterations may suffice to adapt the set of parameters. Such iterative adapting of the parameters of the adapted model is referred to throughout as "iterative detraining" with respect to the undesired training instances.

Generally, a removal request message may be sent for various reasons. For example, a removal request message may represent an absence of consent to further use a training instance, e.g., a withdrawal of consent to use the training instance. This can be the case when the training instance comprises personal information about a certain user. For example, the user itself may send the withdrawal of consent. Such a withdrawal of consent is sometimes also known as a right-to-be-forgotten request or right-to-erasure request. The withdrawal of consent can also be automatic, for example, the user may have provided a conditional consent, e.g., a time-limited consent or a consent dependent on another type of condition, and/or consent may be withdrawn by another party than the user: for example, another party with which a data sharing contract is in place. In these and other cases, the removal request message may be received from a consent management system configured to send the removal request message upon detecting that consent for using a training instance from the training dataset is missing. Such a consent management system can be combined with the system for processing a model, for example, in a single device.

The removal request message does not need to represent an absence of consent to further use the training instance, however. For example, it may be detected, e.g., in an anomaly detection system, that a training instance represents an adversarial instance, sometimes also called poisonous instance. For example, another party may have provided the instance to manipulate the model, e.g., to maliciously sway the model decision boundary. Also in such cases, it is desirable to make the model independent of such adversarial instances. An instance may also be determined to be outdated, for example. In such cases, by making the model independent of undesired training instances, accuracy of the model may be improved. The model may also be made independent from one or more training instances to enable a deployment of the model at a different site, e.g., in a different country. For example, for one or more training instances no consent for processing at the different site may be available, or it may be desired to roll out different versions of the model at different sites, e.g., a free version vs a paid version, etcetera. In such cases, adapted models for respective sites may be determined and provided to one or more respective sites.

The techniques described herein can be used for various types of trained model. In particular, interestingly, the techniques described herein may be applied to non-linear trained models. In various embodiments, the trained model is a classifier, e.g., a model configured to classify input instances into one or more classes. For example, the model may be a binary classifier for two classes or a multiclass classifier for three or more classes. In various embodiments, the trained model is a regression model, for example, a model configured to predict values for one or more output quantities, e.g., a real-valued output, given an input instance. In various embodiments, the trained model is an object detection model, for example, a model configured to detect one or more objects in an input image, e.g., to output a location of an object of a given type detected in the input instance. In various embodiments, the trained model is a segmentation model, e.g., a model configured to associate features of an input instance, e.g., pixels of an input image, with respective class labels. In various embodiments, the trained model is a generative model, e.g., a model configured to generate instances such as images based on latent feature vectors. The model can also be a time-series model, e.g., for time-series modelling or forecasting.

The techniques described herein are applicable to various kinds of data, in particular sensor data such as audio data, image data, video data, radar data, LiDAR data, ultrasonic data, motion data, thermal imaging data, or various individual sensor readings or their histories. For example, in various embodiments, sensor measurements may be obtained from one or more sensors via a sensor interface, e.g., from a camera, radar, LiDAR, ultrasonic, motion, or thermal sensors, or various sensors for measuring physiological parameters such as heart beat or blood pressure, or any combination. Based on these sensor measurements, an input instance may be determined to which the model is applied.

Apart from the embodiments illustrated throughout, various additional embodiments are also envisaged in which the techniques for processing a model as described herein may be advantageously applied.

In an embodiment, the model may be applied in a control system for controlling a computer-controlled machine, e.g., a robot, a vehicle, a domestic appliance, a power tool, a manufacturing machine, a personal assistant, an access control system, etc. The control system may be part of or separate from the computer-controlled machine. For example, a control signal may be determined by the control system based at least in part on an output of the model. As input, the model may obtain data indicative of a state of the computer-controlled machine and/or the physical environment it operates in.

The model may also be applied in various systems for conveying information, e.g., a surveillance system based on images of a building or other object under surveillance, or a medical imaging system, e.g., based on an image of a body or part of it. The model may also be used, for example, in an optical quality inspection system for manufacturing process to inspect manufactured objects for failures. For example, such failures may be detected from images of the manufactured objects.

In an embodiment, the model may be applied in an autonomous vehicle. For example, an input instance may comprise an image of the environment of the vehicle. The model can for example be a classifier, e.g., for classifying traffic signs; a detection or segmentation model, e.g., for detecting or segmenting the image into areas representing a pedestrian, a road surface, another vehicle, etc.; or a time-series forecasting model, e.g., for human trajectory prediction. In various cases, a model output may be used at least in part to control the autonomous vehicle, for example, to operate the autonomous vehicle in a safe mode upon detecting an anomaly, e.g., a pedestrian unexpectedly crossing the road.

In an embodiment, the model may be applied in medical image analysis, e.g., medical image classification. For example, the model may be used to detect a tumour or other object of medical relevance in an image, e.g., a MRI, CT, or PET scan, of a body or part of it, or the model may be used to classify images into different pathologies or other types of medical outcomes.

In an embodiment, the model may be applied for signal processing of measurements of various external devices, e.g., IoT devices. For example, the model may be applied to a streams of incoming sensor measurements of a device, for example, to detect anomalies or other types of events.

In an embodiment, the model may be applied for predictive maintenance, for example to predict whether a component, e.g., a screen or a battery, of a larger device, e.g., a car or a medical device, needs to be replaced based on usage data, e.g., time-series data.

In an embodiment, the model may be used in a system for training an autonomous device such as a robot to interact in a physical environment, for example, in a model used to determine an input to a reinforcement learning system, e.g., by imitation learning. For example, the model may be a feature extractor configured to determine input features for a reinforcement learning system.

In an embodiment, the model may be used to predict various measurable quantities of a physical system, for example, a technical system. Such a system may comprise a device and a physical environment the device interacts with. Generally, various technical systems have an underlying physical model that is too complicated to be modelled explicitly. For example, a model predicting an emission value or other physical quantity of a vehicle engine may depend in a complex, non-linear way on its input parameters, such as speed and load or on the input parameters of the engine control unit (ECU).

Optionally, the training dataset may comprise multiple training instances collected from respective users. Accordingly, the training instances may represent personal information about these users. The removal request message may indicate a user whose training instances are to be removed from the training dataset. For example, records may be stored along with an associated user identifier, the removal request message specifying the user identifier. A removal request message can also indicate the user by specifying the particular records of the user to be removed. Enabling to remove data associated with a particular user may allow to deal appropriately with right-to-erasure requests, also known as right-to-be-forgotten-requests, and/or with users withdrawing consent. A removal request message may indicate data of multiple users.

Optionally, a training instance of a user may comprise one or more sensor measurements of the user. For example, a measurement may be an image of the user, a measurement of a physiological quantity of the user such as a blood pressure or heart rate, etcetera. The measurement can also be a genomic sequence of the user, a fingerprint, and the like. The data may be measured using any appropriate sensor. Since such measured data is intrinsically related to the user, it may be particularly privacy-sensitive and accordingly, being able to remove training instances with such data from a dataset may be particularly desirable.

Optionally, a training instance of a user may be collected by receiving the training instance from a user device. Such a training instance may comprise a sensor measurement by the user device of a physiological quantity of the user, such as a heart rate and/or a blood pressure. For example, the user device may be a smart watch, smart phone, or other kind of wearable device, a home medical measurement device, or the like. The user device may provide the training instance as an instance for which a model output is desired. For example, upon receiving an instance from the user device, the model may be applied to the instance and a model output provided to the user device, the instance being used at a later stage as a training instance to refine the model. Aside from the training instance, also the removal request message may be received from the user device itself, for example, the user may change a setting on the user device to withdraw consent for processing of the measurements of the user device. The removal request message may also be sent by the user from another device, however, e.g., by logging into a user account also used by the user device.

Optionally, the trained model may comprise a feature extractor parametrized by a first set of parameters and a further trained model parametrized by a second set of parameters. In image classification, but also in various other machine learning tasks, the use of a separate feature extractor is known per se. For example, the VGG net trained by the Oxford Visual Geometry Group is used in practice as a feature extractor for various applications. In such cases, the feature extractor and the further trained model may be trained on different datasets. For example, the feature extractor may be a pre-trained feature extractor, e.g., trained on a relatively large dataset, the trained model being obtained by taking the pre-trained feature extractor and just training the further trained model. The feature extractor may be trained by a third party or even be offered as a service to the party applying the trained model, e.g., as part of the AI platforms of Google and Microsoft, and the like. In various cases, the one or more undesired training instances may not be comprised in the further dataset that the feature extractor was trained on. Accordingly, the trained model may be adapted by adapting the second set of parameters of the further trained model but not the first set of parameters of the feature extractor. Accordingly, the use of a separate feature extractor may be beneficial not only because a feature extractor may be used that can be optimized, e.g., trained on a relatively large dataset, and shared among multiple trained models. Indeed, apart from this, it may also allow to relatively easily update the trained model to remove undesired training instances, e.g., fewer parameters may need to be updated and only a part of the model may need to be re-trained, improving efficiency.

Optionally, in one, more, or all iterations of adapting the set of parameters of the adapted model, this adaptation may make use of second derivatives for the objective function with respect to the set of parameters of the adapted model. For example, an iteration of a second-order optimization method such as the Newton method or its variants, may be used to adapt the set of parameters. For example, the Hessian of the objective function may be computed or at least estimated for the current set of parameters. Interestingly, because the set of parameters of the adapted model may be initialized based on the set of parameters of trained model, e.g., set equal to or at least close to the set of parameters of the trained model, this initial estimate can already be relatively good. As the inventors realized, this may enable the use of second-order methods, that may fail to work outside of a relatively small region of the solution but in those cases can converge quicker than first-order methods such as gradient descent.

Optionally, even when second-order methods may be used in some iterations, first-order methods such as gradient descent may be used in other iterations. By flexibly switching between first-order and second-order optimization, e.g., based on which one provides the best improvement to the objective function, the best method for the situation at hand may be flexibly chosen. Accordingly, the advantages of using second-order optimization, e.g., arising due to the good initial estimate for the set of parameters, can be attained when possible while also in other cases, e.g., if second-order optimization turns out to move away from the optimal solution, progress towards determining the adapted model can still be made.

Optionally, when using second-order optimization, diagonal noise may be added to the Hessian to make the Hessian positive semi-definite. This process, known per se in the art as Hessian damping, may be used to avoid problems with the Hessian not being invertible and/or the second-order optimization moving away from the optimal solution. It is noted that Hessian damping does not necessarily involve explicitly computing the Hessian itself, e.g., the optimization may work with an inverse of the Hessian to which diagonal noise has been added. It is also possible to add a regularization term, e.g., an L2 regularization term, on the parameters. Effectively, a regularization term with a certain strength may be regarded as a way of adding diagonal noise of the same size to the diagonal of the Hessian.

Optionally, if the amount of diagonal noise to be added exceeds a certain threshold, the optimization may involve applying one or more first-order optimization iterations, e.g., gradient descent iterations, before again attempting to perform a second-order optimization. For example, in one, two, at most or at least three, or at most or at least five iterations, a first-order optimization iteration may be applied before again determining the Hessian and possibly then again performing a second-order optimization iteration. This way, second-order optimization steps may be avoided in situations where it is likely to give worse results than first-order optimization, e.g., outside of a close neighbourhood of an optimum of the objective function.

Optionally, the second-order optimization may involve determining a product of the inverse of the Hessian for the objective function and the gradient of the objective function. This is the case, for example, in Newton iteration and its variants. In such cases, this product may be determined by minimizing a quadratic equation in the product and the Hessian, as is known per se from Barak A. Pearlmutter, "Fast Exact Multiplication by the Hessian", Neural computation 6(1):147-160, 1994 (incorporated herein by reference). Interestingly, this way, storing and/or inverting the full Hessian may be avoided, improving performance of the second-order optimization iteration.

Optionally, the Hessian matrix used in a second-order optimization iteration or its inverse may be approximated using a quasi-Newton method. Specifically, in an iteration, an initial approximation of the Hessian or its inverse may be determined, and in a following iteration, the approximation to the Hessian or its inverse may be updated, e.g., along with updating the set of parameters. Various quasi-Newton methods can also make sure that the updated Hessian is positive semi-definite, and thus that a second-order optimization step is effective for improving the set of parameters. Accordingly, such methods may avoid to recompute the Hessian matrix for each iteration that needs it, and accordingly further improve performance. Various quasi-Newton methods such as BFGS and L-BFGS are known in the art per se and may be applied here.

Optionally, the trained model may comprise a non-linear model, for example, a neural network. The techniques may be combined with various known neural network architectures, e.g., convolutional neural networks (CNNs), recurrent neural networks (RNNs), networks comprising fully connected layers, or any combination. Neural networks are also known as artificial neural networks. In this case, the set of parameters may comprise weights of nodes of the neural network. For example, the number of layers of the model may be at least 5 or at least 10, and the number of nodes and/or weights may be at least 10000 or at least 100000. Depending on the particular application, various known architectures for neural networks and other types of machine learnable models may be used. Other non-linear models that involve an optimization objective and that can be applied in combination with the techniques herein include various inducing point Gaussian processes, e.g., as known from Titsias, "Variational Model Selection for Sparse Gaussian Process Regression", Technical report, University of Manchester, 2009; Hensman, Fusi and Lawrence, "Gaussian Processes for Big Data", 2013, https://arxiv.org/abs/1309.6835; and Salimbeni and Deisenroth, "Doubly Stochastic Variational Inference for Deep Gaussian Processes", https://arxiv.org/abs/1705.08933, 2017 (all three papers incorporated herein by reference). These non-linear models typically have their sets of parameters determined iteratively so that determining an adapted model iteratively based on a good initial estimate, as described herein, is particularly beneficial.

Optionally, the trained model may comprise a neural network with multiple layers. In such cases, the adapted model may be determined by iteratively adapting weights of only a subset of the multiple layers of the neural network. Generally, any subset may be chosen, e.g., the last $k$ layers or the first $k$ layers for a predefined number $k$; all even layers, etcetera. By considering only a subset of the layers, the performance of the optimization may be greatly improved, while still, an optimum may be determined with respect to a remainder dataset that does not contain the undesired training instances.

Optionally, following the determination of the adapted model, a query instance may be obtained and the adapted model may be applied to the query instance to obtain a model output independent from the one or more undesired training instances. As also discussed elsewhere, the adaptation of the model and the application of the model to query instances may be performed by the same system or different systems. It is also possible for both the adapting and/or the applying to be performed multiple times, for example, in an interleaved fashion in which, at some point after an adapting, an applying is performed, and at some point after the applying, another adapting is performed, etcetera. For example, a system may be configured to obtain multiple respective removal request messages and/or model application messages and to respond to these messages accordingly by adapting or applying the model. Optionally, the party determining the adapted model may have previously trained the model on the training dataset. In such cases, the party may store the training dataset along with the trained model to enable removal request messages to be processed. Accordingly, potentially sensitive information in the training dataset may be kept local to the party performing the training and/or adaptation, for example, whereas the original trained model and its adaptations may be provided to other parties for application to query instances.

Optionally, multiple removal request messages may be received and dealt with in a single operation of determining an adapted model. For example, multiple removal request messages may be collected, e.g., until a certain, preferably rather short, time window has passed, e.g., of at most a minute or at most thirty minutes. Instead or in addition, multiple removal request messages may be collected until a certain maximum amount of undesired training instances is reached, for example, at most ten, at most one hundred, or at most 1% or at most 2% of the training dataset, and/or until a certain maximum amount of messages is received, for example, at most five or at most fifty. By accordingly batching multiple removal request messages, efficiency is improved. However, the total amount of undesired training instances being processed is still preferably kept relatively low to ensure that the current set of parameters of the trained model provide a good estimate for iterative determination of the adapted model.

It will be appreciated by those skilled in the art that two or more of the above-mentioned embodiments, implementations, and/or optional aspects of the invention may be combined in any way deemed useful.

Modifications and variations of any system and/or any computer readable medium, which correspond to the described modifications and variations of a corresponding computer-implemented method, can be carried out by a person skilled in the art on the basis of the present description, and similarly, for modifications and variations of a method or medium based on described modifications and variations of a system.

## BRIEF DESCRIPTION OF THE DRAWINGS

These and other aspects of the invention will be apparent from and elucidated further with reference to the embodiments described by way of example in the following description and with reference to the accompanying drawings, in which:

Fig. 1 shows a system for processing a model trained based on a loss function;
Fig. 2 shows a detailed example of how to adapt a trained model for one or more undesired training instances, and how to apply the adapted trained model to an input instance;
Fig. 3 shows a detailed example of how to adapt a trained model comprising a feature extractor;
Fig. 4 shows a computer-implemented method of processing a model trained based on a loss function;
Fig. 5 shows a computer-readable medium comprising data.

It should be noted that the figures are purely diagrammatic and not drawn to scale. In the figures, elements which correspond to elements already described may have the same reference numerals.

## DETAILED DESCRIPTION OF EMBODIMENTS

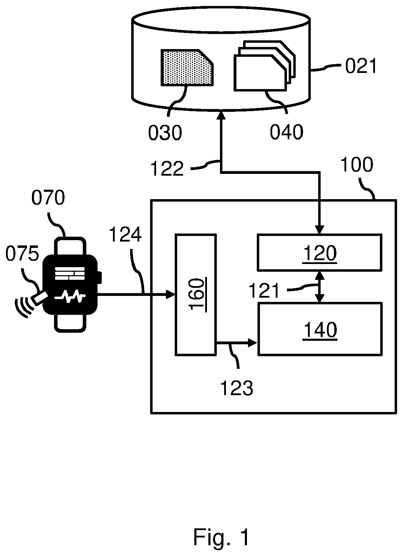

**Fig. 1** shows a system 100 for processing a model trained based on a loss function. The model may be configured to provide a model output given an input instance. The model may be trained on a training dataset by iteratively optimizing an objective function. The objective function may include respective losses according to the loss function for respective training instances of the training dataset. The system 100 may comprise a data interface 120 and a processor subsystem 140 which may internally communicate via data communication 121. Data interface 120 may be for accessing the model 030 and the training dataset 040 on which the model has been trained.

The processor subsystem 140 may be configured to, during operation of the system 100 and using the data interface 120, access data 030, 040. For example, as shown in Fig. 1, the data interface 120 may provide access 122 to an external data storage 021 which may comprise said data 030, 040. Alternatively, the data 030, 040 may be accessed from an internal data storage which is part of the system 100. Alternatively, the data 030, 040 may be received via a network from another entity. In general, the data interface 120 may take various forms, such as a network interface to a local or wide area network, e.g., the Internet, a storage interface to an internal or external data storage, etc. The data storage 021 may take any known and suitable form.

System 100 may also comprise a removal request interface 160 configured for receiving a removal request message 124. The removal request message 124 may identify one or more undesired training instances of the training dataset. Removal request interface 160 may internally communicate with processor subsystem 140 via data communication 123. Removal request interface 160 may be arranged for direct communication with other systems from which removal request messages may be received, e.g., user devices, e.g., using USB, IEEE 1394, or similar interfaces. Removal request interface 160 may also communicate over a computer network, for example, a wireless personal area network, an internet, an intranet, a LAN, a WLAN, etc. For instance, removal request interface 160 may comprise a connector, e.g., a wireless connector, an Ethernet connector, a Wi-Fi, 4G or 4G antenna, a ZigBee chip, etc., as appropriate for the computer network. The figure shows a removal request message 124 being received from smart watch 070, for example via the internet, where the smart watch 070 is also configured to measure one or more physiological quantities of the user using one or more sensors, such as sensor 075 shown in the figure. System 100 may form a user data processing system together with one or more user devices 070 and/or other systems that apply the model.

Removal request interface 160 may also be an internal communication interface, e.g., a bus, an API, a storage interface, etc. For example, system 100 may be part of a consent management system configured to ensure that consent is available for the training dataset 040; for example, another part of the consent management system may send a removal request message to system 100 as described herein. As another example, system 100 may be part of an anomaly detection system configured to detect and deal with undesired training instances, e.g., adversarial examples or other types of outliers, in which case another part of the anomaly detection system may send a removal request message to system 100 as described herein.

Processor subsystem 140 may be configured to, during operation of the system 100 and using the data interface 120, upon receiving the removal request message 124, make the model independent from the one or more undesired training instances. To make the model independent, processor subsystem 140 may be configured to remove the one or more undesired training instances from the training dataset to obtain a remainder dataset, and to an adapted model for the remainder dataset. To determine the adapted model for the remainder dataset, processor subsystem 140 may be configured to initialize a set of parameters of the adapted model based on the set of parameters of the trained model, and to iteratively adapt the set of parameters of the adapted model by optimizing the objective function with respect to the remainder dataset.

As an optional component, the system 100 may comprise an image input interface or any other type of input interface (not shown) for obtaining sensor data from a sensor, such as a camera. Processor subsystem 140 may be configured to obtain an input instance for the trained model based on the obtained sensor data, and to apply the adapted model to the obtained input instance. For example, the camera may be configured to capture image data, processor subsystem 140 being configured to determine an input instance from the image data. The input interface may be configured for various types of sensor signals, e.g., video signals, radar/LiDAR signals, ultrasonic signals, etc. As an optional component, the system 100 may also comprise a display output interface or any other type of output interface (not shown) for outputting an output of the adapted model for an input instance to a rendering device, such as a display. For example, the display output interface may generate display data for the display which causes the display to render the model output in a sensory perceptible manner, e.g., as an on-screen visualisation. As an optional component, the system 100 may also comprise an actuator interface (not shown) for providing, to an actuator, actuator data causing the actuator to effect an action in an environment of system based on a model output determined for an input instance.

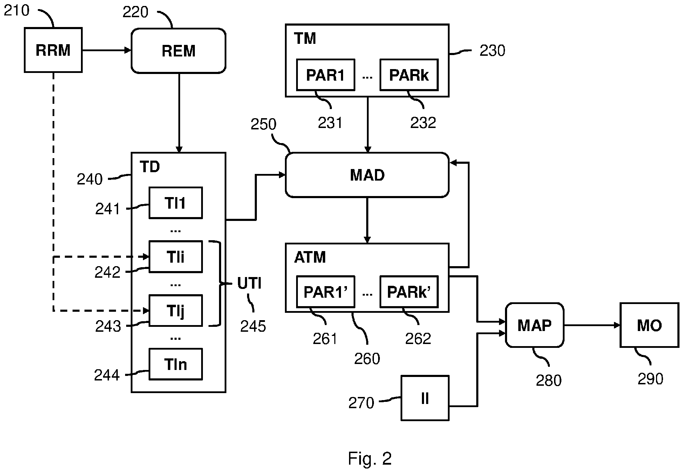

Various details and aspects of the operation of the system 100 will be further elucidated with reference to Figs. 2-3, including optional aspects thereof.

In general, the system 100 may be embodied as, or in, a single device or apparatus, such as a workstation, e.g., laptop or desktop-based, or a server. The device or apparatus may comprise one or more microprocessors which execute appropriate software. For example, the processor subsystem may be embodied by a single Central Processing Unit (CPU), but also by a combination or system of such CPUs and/or other types of processing units. The software may have been downloaded and/or stored in a corresponding memory, e.g., a volatile memory such as RAM or a non-volatile memory such as Flash. Alternatively, the functional units of the system, e.g., the data interface and the processor subsystem, may be implemented in the device or apparatus in the form of programmable logic, e.g., as a Field-Programmable Gate Array (FPGA) and/or a Graphics Processing Unit (GPU). In general, each functional unit of the system may be implemented in the form of a circuit. It is noted that the system 100 may also be implemented in a distributed manner, e.g., involving different devices or apparatuses, such as distributed servers, e.g., in the form of cloud computing.

**Fig. 2** shows a detailed yet non-limiting example of how to adapt a trained model for one or more undesired training instances, and how to apply the adapted trained model to an input instance.

Shown in the figure is a trained model **TM**, 230, as well as a training dataset **TD**, 240, on which the model has been trained. The trained model may be configured to provide a model output given an input instance. The trained model may have been trained on training dataset **TD** using techniques known from the art. It is also possible that trained model **TM** and training dataset **TD** have been determined as described herein, e.g., as a remainder dataset and corresponding adapted model determined by removing one or more previous undesired training instances from an earlier dataset. Trained model **TM** may be a non-linear model, e.g., a neural network. Generally, trained model **TM** may have been trained using supervised learning, e.g., using a training dataset **TD** of training instances with associated desired outcomes of the trained model. For example, trained model **TM** may be a regression or classification model. It is also possible for the trained model or at least parts of the trained model to be trained using unsupervised learning e.g., word2vec-style embeddings or self-supervised learning, e.g., image rotation prediction tasks, however. Such unsupervised and/or self-supervised modules can also be detrained using the techniques described herein. For ease of exposition, iterative detraining of a trained model **TM** trained by supervised learning is discussed. Shown in the figure are several training instances **TI1**, 241; **TIi**, 242; **TIj**, 243; and **TIn**, 244. Generally, the number of training instances may be at least or at most 10000, at least or at most 100000, or at least or at most 1000000, for example.

Mathematically, training of trained model **TM** on training dataset **TD**, in this case in a supervised learning setting, may be formulated as follows. Training dataset **TD** may be denoted $D_{train} = \{u_i\}_{i=1}^{n}$ , where a training instance **TI\*** may be denoted $u_i = (x_i, y_i)$. For example, an input instance may comprise an input feature vector $x_i \in X$, e.g.,

$$\mathcal{X} = \mathbb{R}^d,$$

and a target value

$$y_i \in \mathcal{Y}.$$

For example, **TM** may be a classification model, in which case $\mathcal{Y}$ may equal {0,1, ..., $C$ - 1} with $C$ the total number of classes. As another example, **TM** may be a regression model, in case $\mathcal{Y}$ may equal $\mathbb{R}$ , for example. Generally, trained model **TM** may be trained on the training dataset **TD** to learn a function

$$f: \mathcal{X} \to \mathcal{Y}$$

that generalizes from the trianing dataset **TD** to unseen input instances.

Generally, training dataset **TD** may comprise one or more sensor measurements of a user, for example, an image represented by pixels, features, or the like, or measurements of various physiological quantities, e.g., in a time series, etcetera.

Trained model **TM** may have been trained on training dataset **TD** based on a loss function. For example, trained model **TM** may be an Empirical Risk Minimization, ERM, model. Examples of such models include regularized least square regression models, logistic regression models, and various types of neural networks such as deep neural networks and sparse deep/non-deep Gaussian Process models. As shown in the figure, such a model is typically parametrized by a set of parameters $\theta$, such as parameters **PAR1**, 231, up to **PARk**, 232, shown in the figure. For example, the set of parameters may be at most or at least 1000, at most or at least 10000, or at most or at least 1000000.

For example, in case trained model **TM** is a regression model, the model output of **TM** for an input instance $x$ may be denoted $f(x; \theta)$. As another example, in case trained model **TM** is a binary classification model, the model output of **TM** for an input instance $x$ may be given as $f(x) = \mathbb{I}(f(x; \theta) > 0)$, and similarly for multi-class classification. The loss function may indicate a loss for a respective training instance, for example, the loss function may determine a deviation of the model output for the training instance to a desired model output. The model **TM** may have been trained by optimizing an objective function including respective losses according to the loss function for respective training instances of the training dataset **TD.** The combination, e.g., sum, of the respective losses is sometimes referred to as the empirical risk over the training dataset **TD.** For example, the set of parameters **PAR\*** may have been determined by solving the following optimization problem:

$$\boldsymbol{\theta}_{opt} = \operatorname{argmin} R(\boldsymbol{\theta})$$

where $R(\boldsymbol{\theta}) = \frac{1}{n} \sum_{x,y \in D_{train}} l(\boldsymbol{x}, y, \boldsymbol{\theta})$ combines repective losses for the training dataset **TD** in terms of a loss function

$$l: \mathbb{R}^d \times \mathcal{Y} \times \mathbb{R}^k \to \mathbb{R},$$

e.g., squared loss, cross-entropy loss, etcetera. The objective function may include additional terms, such as regularizers, etc. The model may have been trained on a training dataset in a conventional way, for example, by iterative optimization methods such as gradient descent, e.g., stochastic, batch or mini-batch gradient descent.

It is noted that, in order to apply trained model **TM** to an input instance, it is typically not needed to access training dataset **TD.** For example, for a non-kernelized SVM classifier, a model output in the form of a prediction may be determined as $f(\boldsymbol{x}) = \mathbb{I}[\boldsymbol{\theta}^T \boldsymbol{x} > 0]$, where $\theta$ is a vector specifying the decision hyperplane in the input feature space comprised in the set of parameters **PAR\***.

Interestingly, however, training dataset **TD** may still be stored or accessed along with the trained model in order to deal with removal request messages. The figure shows a removal request message **RRM**, 210. A removal request message may identify one or more undesired training instances **UTI**, 245, of the training dataset **TD.** For example, removal request message **RRM** shown in the figure indicates training instances **TIi,** 242 up to **TIj,** 243. The undesired training instances may be indicated in various ways, e.g., by including the instances in the message, or by including indices in the message. As another example, the training dataset **TD** may comprise multiple training instances collected from respective users, in which case removal request message **RRM** may indicate a user whose training instances are to be removed from the training dataset **TD,** for example, in the form of a user identifier or the like.

Upon receiving the removal request message **RRM**, trained model **TM** may be made independent from the one or more undesired training instances **UTI.** To this end, in a removal operation **REM**, 220, the one or more undesired training instances **UTI** may be removed from the training dataset **TD,** thus obtaining a remainder dataset. As shown in this figure, this operation is typically performed in-place on the training dataset **TD,** although it is also possible to make a copy of the training dataset without the undesired training instances.

Further, in a model adaptation operation **MAD**, 250, an adapted model **ATM**, 260, for the remainder dataset may be determined. Mathematically speaking, denoting an undesired training instance as *u' = (x',y')* the problem of determining an adapted model ATM with respect to this undesired training instance may be phrased as the problem of determining an adapted model that is trained on the remainder dataset $D'_{train} = D_{train} - \{u'\}$, in the sense that the adapted model could be obtained by training from scratch from the remainder dataset, or at least as a result of an optimization with respect to the remainder dataset. The above definition of $D'_{train}$ demonstrates the case of a single undesired training instance. The case of multiple undesired training instances can be handled, for example, by repeating the removal of single instances, or by performing an optimization with respect to a remainder dataset $D'_{train}$ from which the multiple instances are removed. Typically, the adapted model **ATM** has the same structure as the original trained model **TM,** e.g., the same function or procedure may be used to determine the model output in the adapted model **ATM** as in the trained model **TM,** but based on a different values for the parameters. Shown in the figure are parameters **PAR1'**, 261, up to **PARk'**, 262, of the adapted model corresponding to parameters **PAR1** up to **PARk** of the trained model **TM,** respectively. The number of parameters **PAR\*** and **PAR\*'** is typically the same.

Interestingly, in order to determine the parameters **PAR\*'** of the adapted model **ATM,** these parameters may first be initialized based on the set of parameters **PAR\*** of the original trained model **TM,** e.g., be set equal to it, or equal up to a small amount of added noise, etcetera. The parameters **PAR\*'** may then be iteratively adapted by optimizing the same the objective function that was used to train the trained model **TM,** but now with respect to the remainder dataset. For example, in terms of the above mathematical phrasing of the optimization, the parameters **PAR\*'** of the adapted model may be denoted $\theta_{opt}'$ and determined as an optimization:

$$\boldsymbol{\theta}'_{opt} = \operatorname{argmin} R'(\theta) = \operatorname{argmin} \frac{1}{n}\textstyle\sum_{(x,y)\in D'_{train}} l(\boldsymbol{x}, y, \boldsymbol{\theta}).$$

Generally, various ways of iteratively adapting a set of parameters of a model are known in the art per se and can be applied here, for example, stochastic approaches such as stochastic gradient descent. For example, the Adam optimizer, as disclosed in Kingma and Ba, "Adam: A Method for Stochastic Optimization" (available at https://arxiv.org/abs/1412.6980 and incorporated herein by reference) may be used. As is known and as also applies to the training of the original trained model **TM**, such optimization methods may be heuristic and/or arrive at a local optimum. Training may be performed on an instance-by-instance basis or in batches, e.g., of at most or at least 64 or at most or at least 256 instances. Various advantageous alternatives and extensions to gradient descent-type methods are discussed throughout. The iterative adaptation of the set of parameters **PAR\*'** may comprise performing at most a predefined number of iterations and/or terminating the iterative adaptation based on a stopping condition, e.g., if a change in objective function is smaller than a predefined threshold in one or more subsequent iterations, or using other stopping conditions known per se. In some cases, the use of first-order optimization may be preferred, e.g., because of generally requiring less memory than second-order optimization methods.

Interestingly, the inventors realized that the iterative adaptation of the set of parameters **PAR\*'** may be improved by, in an iteration of the iterative adapting of the set of parameters of the adapted model, using second derivatives for the objective function. Generally, the use of second derivatives in optimization may be referred to as a second-order optimization, as opposed to first-order optimization, for example gradient descent, that does not use second derivatives. Specifically, because a good initial estimate of the set of parameters **PAR\*'** may be obtained based on the set of parameters **PAR\*** of the original trained model, the use of a second-order optimization method may be particularly effective. The second derivatives may be evaluated per iteration, but it is also possible to use a quasi-Newton method to keep track of the second derivatives, as discussed further below.

Generally, during the iterative optimization, first-order and second-order optimization steps may be combined, e.g., in one or more iterations a first-order optimization step may be applied whereas in one or more other iterations, a second-order optimization step may be applied. By choosing appropriate optimization steps for respective iterations, overall efficiency of the optimization may be improved. In fact, it may be not even be possible to reach an optimum by just second-order optimization steps, for example, if the initial estimate for the set of parameters **PAR\*'** is insufficiently accurate, in which performing one or more first-order optimization steps in addition may help to reach an optimum at all. In particularly beneficial embodiments, a second-order optimization step may be performed if this is expected to provide a substantially better result than a first-order optimization step, for example, if a sufficient condition for the second-order optimization step improving the objective function is satisfied and/or a sufficient criterion indicating that a second-order step can provide a substantially better result than a first-order step is satisfied. One or more first-order optimization steps may be performed otherwise. For example, the sufficient condition may be faster to check than performing the second-order iteration itself, in which case a particular performance improvement may be achieved. Various examples are provided throughout.

Specifically, in some or all iterations, a Newton iteration step may be used to update the set of parameters. In particular, in terms of the mathematical formulation presented above, it may be noted that by the definition of first-order optimality, $\nabla R'\left(\boldsymbol{\theta}'_{opt}\right) = 0$. Accordingly, in this case, a Taylor series expansion of $\nabla R'(\theta)$ around $\theta_{opt}$ may be obtained as:

$$0 = \nabla R'\left(\boldsymbol{\theta}_{opt}\right) + \left(\boldsymbol{\theta} - \boldsymbol{\theta}_{opt}\right)\nabla^2 R'\left(\boldsymbol{\theta}_{opt}\right)$$

$$\boldsymbol{\theta} = \boldsymbol{\theta}_{opt} - H_{\boldsymbol{\theta}_{opt}}^{-1}\nabla R'(\boldsymbol{\theta}opt)$$

by noting that $H_{\boldsymbol{\theta}_{opt}}^{-1} = \nabla^{-2}R'\left(\boldsymbol{\theta}_{opt}\right)$.

In some exceptional cases, the above equation may converge in a single step from the initial set of parameters, e.g., $\theta_{opt}$ to $\theta_{opt}'$. For example, this may be the case if $R'(\theta)$ is quadratic at $\theta_{opt}$, e.g., if the approximation of $R'(\theta)$ through the first two terms of the Taylor series expansion is exact. In many cases, however, a performing a single iteration will not arrive at an optimum, and accordingly, multiple iterations may be performed to reach such an optimum, e.g., a local optimum, of the objective function. For example, multiple successive Newton steps may be performed. Respective step sizes for the Newton iterations can be determined, for example, via Wolfe or Goldstein conditions as known per se.

It may occur that, in at least one second-order iteration, the Hessian matrix is not positive definite. As a consequence, the Hessian matrix may not be invertible, or the Hessian matrix may be invertible but a second-order iteration may move away from an optimum of the objective function. Generally, this may happen if the optimum $\theta_{opt}'$ for the remainder dataset is relatively far away from the values $\theta_{opt}$ to which the set of parameters of the adapted model are initialized. In order to prevent this, in one or more second-order optimization iterations, diagonal noise may be added to the Hessian, for example, by a process that is known per se as Hessian damping. For example, when applying Hessian damping, an increasing amount of diagonal noise may be added to the Hessian, e.g., $H = H + \tau I$, with $\tau \in \mathbb{R}^+$. For example, diagonal noise may be added until the Hessian $H$ becomes diagonally dominant with all the diagonal values being positive, and as a result, may become a positive definite matrix. As also discussed elsewhere, diagonal noise may be added without explicitly computing the Hessian, e.g., by including a regularization term, such as a L2 regularization term, to the loss function. For example, a regularization term with a given strength $\tau$ may be used to effectively add $\tau$-sized diagonal noise to the Hessian.

Interestingly, the inventors realized that, in order to determine the amount of diagonal noise to be added to the Hessian, it may not be needed to check for positive definiteness via a Cholesky decomposition, as sometimes performed in the art, are available. Such a positive definiteness check may incur a $O(nd^3)$ worst-case time complexity, where $d$ is the number of parameters of the model and $n$ is the number of training instances. As the inventors realized, this performance penalty may be avoided, for example, by checking if a sufficient amount of diagonal noise is added by checking if the second-order optimization leads to a descent direction of the objective function, e.g., by checking if

$$\nabla R'\left(\boldsymbol{\theta}_{opt}\right)\boldsymbol{H}_{\boldsymbol{\theta}_{opt}}^{-1}\nabla R'\left(\boldsymbol{\theta}_{opt}\right) < 0$$

in the case of a Newton iteration. This check may be performed, e.g., in $O(nd)$ when using stochastic Hessian approximation as discussed further elsewhere, or in $O(ndm)$ with $m \ll d$ when using the Conjugate Gradient Hessian vector product technique discussed further below. Here $n$ refers to the number of training datapoints. Accordingly, in various settings this may be more efficient than using the Cholesky decomposition. As another example, checking if a sufficient amount of diagonal noise is added may be performed by checking if the Hessian matrix is a diagonally dominant symmetric matrix with all positive diagonal values. This check may be performed in $O(nd^2)$ time complexity by materializing the Hessian, and may optionally be performed using early abandoning, e.g., constructing the Hessian one data point at a time, e.g., at a cost of $O(d^2)$, and stopping the check as soon as the condition is violated. Generally, by checking a sufficient but not necessary condition for positive definiteness instead of checking for positive definiteness directly, for various parameter values, overall performance may be improved.

In some embodiments, after determining a Hessian for the objective function with respect to set of parameters **PAR\*'**, an amount of diagonal noise may be determined needed to make the Hessian positive definite. For example, a sufficient condition for positive definiteness such as one of the conditions above may be used to check if an amount of noise is sufficient. If the amount of diagonal noise to be added exceeds a threshold, the set of parameters **PAR\*'** may be adapted using a first-order optimization method, e.g., gradient descent optionally with an appropriate step size selection mechanism such as line-search, in one or more iterations. For example, checking if the amount of diagonal noise exceeds the threshold may comprise checking if the amount of diagonal noise is bigger than a fixed threshold, e.g., 10 or 100 times each the row sum of the Hessian matrix. Accordingly, it may be avoided to use second-order methods in settings where they may lead the optimization in a counterproductive direction, e.g., when relatively far away from a local optimum, and instead, first-order methods may be used in this case. Afterwards, again a Hessian may be determined based on which, for example, a second-order optimization step may be applied if possible.

Various second-order optimization methods, for example Newton iteration, may involve computing and inverting the Hessian matrix. Using known algorithms, this may be performed in $O(Nd^3)$ operations in an iteration, where $d$ is the number of rows and columns of the Hessian matrix, e.g., the size of the vector $\theta$. As the inventors realized, this can be undesirable in the setting of determining an adapted model for a remainder dataset since the set of parameters **PAR\*'** may be quite large, e.g., at least 100000, at least 1 million or even at least 10 million.

Interestingly, in various embodiments, a product of the inverse of the Hessian for the objective function and the gradient of the objective function, as used e.g. in a Newton iteration, may be determined by minimizing a quadratic equation in the product and the Hessian. Accordingly, the $O(nd^3)$ time complexity may be reduced to around $O(ndm)$ with $m \ll d$, which is particularly beneficial in the present setting in which the number of parameters is relatively large. For example, the Conjugate Gradient Hessian vector product technique as disclosed in Barak Pearlmutter, "Fast exact multiplication by the Hessian", Neural Computation, 6(1):147-160, 1994 (incorporated herein by reference insofar as this technique is concerned) may be used. In various embodiments, the product of the inverse of the Hessian with the gradient vector, as used e.g. in Newton iteration, may also be estimated stochastically, e.g., as known per se from P. Koh and P. Liang, "Understanding black-box predictions via influence functions", Proceedings of ICML 2017 (section 3 on stochastic Hessian estimation being incorporated herein by reference). Also stochastic Hessian estimation can significantly improve performance, e.g., allowing an iteration to be performed in $O(nd)$ time.

In various embodiments, the Hessian matrix or its inverse for use in a second-order iteration may be determined by estimating it using a quasi-Newton method. In such cases, in an iteration, a current approximation of the Hessian matrix may be estimated by adapting a previous approximation of the Hessian matrix, or similarly for its inverse. Various such methods, e.g., BFGS or L-BFGS, are known per se and can be applied.

In various embodiments, an adapted model for the remainder dataset may be determined by iteratively adapting only a subset of the set of parameters of the adapted model **ATM**. For example, the other parameters, e.g., including parameters that have originally been trained on the trained model including the undesired training instances, may be copied from the original trained model **TM**. Specifically, in the case of a neural network with multiple layers, weights of only a subset of the multiple layers of the neural network may be adapted in the optimization. Interestingly, still, the undesired training instances **UTI** may be considered to be sufficiently removed, while the optimization problem may be solved considerably more efficient by optimizing only a subset of the set of parameters.

Having determined adapted model **ATM**, as shown in the figure, a model application operation **MAP**, 280, may be used to apply the adapted model **ATM** to an input instance **II**, 270, resulting in a model output **MO**, 290. For example, model application **MAP** may be performed by the same system that determined the adapted model or by another system that obtains the adapted model. Interestingly, the model output **MO** may be considered to be independent of the undesired training instances **UTI** at least in the sense that its set of parameters **PAR*'** may represent an optimum of an objective function defined with respect to a remainder dataset from which the undesired training instances **UTI** have been removed. Moreover, also the remainder dataset itself and the adapted model **ATM** may in that sense be considered independent of the undesired training instances **UTI**. Accordingly, an appropriate way of dealing with removal request message **RRM** is shown.

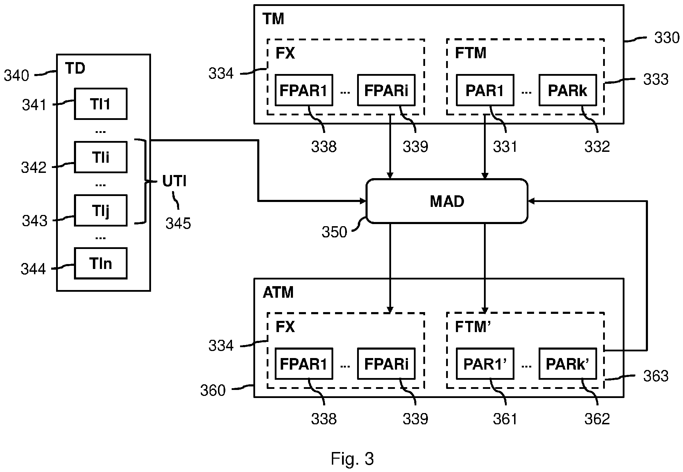

**Fig. 3** shows a detailed, yet non-limiting, example of how to adapt a trained model comprising a feature extractor. This example may be based on the example of Fig. 2. Shown in the figure is a trained model **TM**, 330, configured to provide a model output given an input instance. Trained model **TM** may be trained on a training dataset **TD**, 340, by iteratively optimizing an objective function including respective losses according to the loss function for respective training instances of the training dataset. For example, shown in the figure are training instances **TI1**, 341; **TIi**, 342; **TIj**, 343 and **TIn**, 344. Similarly to Fig. 2, one or more training instances may be identified as undesired training instances from which the trained model **TM** is to be made independent. By way of example, the figure shows training instances **TIi** and **TIj** being identified as undesired training instances **UTI**, 345. Accordingly, a model adaptation operation **MAD**, 350, may be performed to determine an adapted model **ATM**, 360 for the remainder dataset obtained by removing the undesired training instances **UTI** from the training dataset **TD**.

Interestingly, in the example shown in this figure, trained model **TM** may comprise a feature extractor **FX**, 334, parametrized by a first set of parameters **FPAR1**, 338, up to **FPARi**, 339. Moreover, trained model **TM** may comprise a further trained model **FTM**, 333, parametrized by a second set of parameters **PAR1**, 331, up to **PARk**, 332. Accordingly, trained model **TM** may be applied to a query instance by applying the feature extractor **FX** to the query instance to obtain a feature representation of the query instance, and applying the further trained model **FTM** to the feature representation to obtain a model output.

The further trained model **FTM** may be trained on the training dataset **TD** including the undesired training instances **UTI**. The trained model **TM** being trained based on a loss function may in such an example refer to at least its further trained model **FTM** being trained based on a loss function, e.g. by iteratively updating an objective function including respective losses according to the loss function for training instances of the training dataset, inputs to the further trained model **FTM** in this case being given by the feature extractor **FX**.

Interestingly, however, the feature extractor **FX** may be trained on a further dataset (not shown) that does not include the undesired training instances. For example, the feature extractor may be a pre-trained feature extractor, for example, obtained from a third party. Although the feature extractor is illustrated in the figure as comprising the set of parameters, it will be understood that the feature extractor **FX** may be an external feature extractor, e.g., accessed via an API, e.g., of a machine learning framework such as the Google AI Platform or the Microsoft AI Platform. Generally, the feature extractor **FX** may be shared among multiple trained models. Also, the feature extractor **FX** may be trained on a relatively large dataset, for example, of publicly available data, whereas the further trained model **FTM** may be trained on a smaller dataset. For example, the feature extractor may be the VGG network of Oxford University or a similar general pre-trained model. Although the feature extractor may be trained using a loss function, this is not needed and various other ways of training feature extractors that are known may be used, or may have been used by the third party training the feature extractor **FX**.

Interestingly, by using a general feature extractor **FX** trained on a relatively large dataset, a smaller dataset may suffice for training the further trained model **FTM**. For example, the training dataset **TD** may comprise at most 100, at most 1000 or at most 10000 training instances. On the other hand, the training dataset of the feature extractor may comprise at least 100000 or at least 1000000 training instances, for example. Although using a relatively small dataset for training the further trained model may be beneficial from a performance and data collection effort point of view, this may also make it particularly relevant to properly deal with removal request messages, e.g., since a single instance of the training dataset **TD** may have a relatively greater influence on the parameters **PAR\*** and/or model outputs of the further trained model **FTM.**

When determining adapted model **ATM**, parameters **FPAR\*** of the feature extractor **FX** may be kept unchanged. For example, as shown in the figure, adapted model **ATM** may comprise the same feature extractor **FX** as trained model **TM**, and also the set of parameters **PAR1, ..., PARi** of the feature extractor of the trained model may be used. For example, in case trained model **TM** is adapted in-place, no adaptations to this part of the trained model may be needed. Still, this part of the model may be independent of the undesired training instances **UTI.**

As shown in the figure, however, adapting the trained model **TM** may comprise adapting the further trained model **FTM**, obtaining an adapted further trained model **FTM'**, 363. Shown in the figure are parameters **PAR1'**, 361, up to **PARk'**, 362, of the further trained model **FTM'** of the adapted model **ATM**. These parameters may be adapted as described for the trained model of Fig. 2. For example, parameters **PAR\*'** may be initialized based on the set of parameters **PAR\*** from the trained model **TM**, for example, as done in Fig. 2. The set of parameters **PAR\*'** of the further trained model **FTM'** may then be iteratively adapted by optimizing an objective function with respect to the remainder dataset obtained by removing the undesired training instances **UTI** from the training dataset **TD.** Various techniques for optimizing the objective function discussed for Fig. 2 may be applied here. Interestingly, the further trained model **FTM** may be smaller than a full trained model for the same task and/or may be trained on a smaller dataset. As a consequence, iterations of the iterative optimization may be faster, and moreover, fewer iterations may be needed to reach an optimum. Accordingly, performance may be improved while still determining a model that is independent from the undesired training instances **UTI.**

Although not shown in the figure, adapted trained model **ATM** may be applied to a query instance by applying the feature extractor **FX** of the adapted trained model **ATM**, for example, the original feature extractor **FX** of the trained model **TM**, to the query instance to obtain a feature representation of the query instance; and applying the adapted further trained model **FTM'** to the feature representation to obtain a model output.

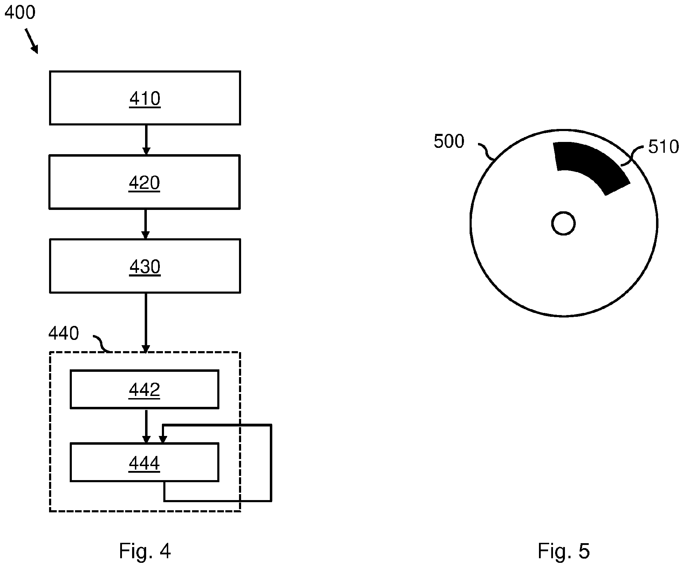

**Fig. 4** shows a block-diagram of computer-implemented method 400 of processing a model trained based on a loss function. The model may be configured to provide a model output given an input instance. The model may be trained on a training dataset by iteratively optimizing an objective function. The objective function may include respective losses according to the loss function for respective training instances of the training dataset. The method 400 may correspond to an operation of the system 100 of Fig. 1. However, this is not a limitation, in that the method 400 may also be performed using another system, apparatus or device.

The method 400 may comprise, in an operation titled "ACCESSING MODEL, TRAINING DATA", accessing 410 the model and the training dataset on which the model has been trained.

The method 400 may further comprise, in an operation titled "RECEIVING REMOVAL REQUEST MESSAGE", receiving 420 a removal request message. The removal request message may identify one or more undesired training instances of the training dataset.

The method 400 may further comprise, upon receiving the removal request message, making the model independent from the one or more undesired training instances. In order to make the model independent from the one or more undesired training instances, the method 400 may comprise, in an operation titled "REMOVING UNDESIRED TRAINING INSTANCES", removing 430 the one or more undesired training instances from the training dataset to obtain a remainder dataset.

To make the model independent, the method 400 may further comprise, in an operation titled "DETERMINING ADAPTED MODEL", determining 440 an adapted model for the remainder dataset. To determine the adapted model, the method 400 may comprise, in an operation titled "INITIALIZING PARAMETERS", initializing 442 a set of parameters of the adapted model based on the set of parameters of the trained model. To determine the adapted model, the method 400 may further comprise, in an operation titled "ITERATIVELY ADAPTING WITH RESPECT TO REMAINDER DATASET", iteratively adapting 444 the set of parameters of the adapted model by optimizing the objective function with respect to the remainder dataset.

It will be appreciated that, in general, the operations of method 400 of Fig. 4 may be performed in any suitable order, e.g., consecutively, simultaneously, or a combination thereof, subject to, where applicable, a particular order being necessitated, e.g., by input/output relations.

The method(s) may be implemented on a computer as a computer implemented method, as dedicated hardware, or as a combination of both. As also illustrated in **Fig. 5**, instructions for the computer, e.g., executable code, may be stored on a computer readable medium 500, e.g., in the form of a series 510 of machine-readable physical marks and/or as a series of elements having different electrical, e.g., magnetic, or optical properties or values. The executable code may be stored in a transitory or non-transitory manner. Examples of computer readable mediums include memory devices, optical storage devices, integrated circuits, servers, online software, etc. Fig. 5 shows an optical disc 500.

Examples, embodiments or optional features, whether indicated as non-limiting or not, are not to be understood as limiting the invention as claimed.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of" when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

**Claims**

1. A system (100) for processing a model trained based on a loss function, the model being configured to provide a model output given an input instance, the model being trained on a training dataset by iteratively optimizing an objective function, the objective function including respective losses according to the loss function for respective training instances of the training dataset, the system comprising:

   - a data interface (120) configured for accessing the model (030) and the training dataset (040) on which the model has been trained;
   - a removal request interface (160) configured for receiving a removal request message, the removal request message identifying one or more undesired training instances of the training dataset;
   - a processor subsystem (140) configured to, upon receiving the removal request message, make the model independent from the one or more undesired training instances by:

     - removing the one or more undesired training instances from the training dataset to obtain a remainder dataset;
     - determining an adapted model for the remainder dataset by:

       - initializing a set of parameters of the adapted model based on the set of parameters of the trained model;
       - iteratively adapting the set of parameters of the adapted model by optimizing the objective function with respect to the remainder dataset.

2. The system (100) of claim 1, wherein the training dataset comprises multiple training instances collected from respective users, the removal request message indicating a user whose training instances are to be removed from the training dataset.

3. The system (100) of claim 2, wherein a training instance of a user comprises one or more sensor measurements of the user, for example, an image.

4. The system (100) of claim 3, wherein the processor subsystem (140) is further configured to collect the training instance of the user by receiving the training instance from a user device, the training instance comprising a sensor measurement by the user device of a physiological quantity of the user.

5. The system (100) of any one of the preceding claims, wherein the trained model comprises a feature extractor parametrized by a first set of parameters and a further trained model parametrized by a second set of parameters, the feature extractor being trained on a further dataset not comprising the undesired training instances, the processor subsystem (140) being configured to determine the adapted model by adapting the second set of parameters.

6. The system (100) of any one of the preceding claims, wherein the processor subsystem (140) is configured to, in an iteration of the iterative adapting of the set of parameters of the adapted model, determine one or more second derivatives for the objective function with respect to said set of parameters and adapting said set of parameters based on the determined second derivatives.

7. The system (100) of claim 6, wherein the processor subsystem (140) is configured to, in a further iteration of the iterative adapting of the set of parameters of the adapted model, adapt said set of parameters using a first-order optimization method.

8. The system (100) of claim 7, wherein the processor subsystem (140) is further configured to:

    - determine an amount of diagonal noise to be added to make a Hessian for the objective function with respect to said set of parameters positive definite; and
    - if the amount of diagonal noise to be added exceeds a threshold, adapt said set of parameters using the first-order optimization method in one or more iterations.

9. The system (100) of any one of claims 6 to 8, wherein the processor subsystem (140) is configured to determine a product of the inverse of the Hessian for the objective function and the gradient of the objective function by minimizing a quadratic equation in the product and the Hessian.

10. The system (100) of claim 6 or 7, wherein the processor subsystem (140) is configured to approximate a Hessian matrix or an inverse of a Hessian matrix using a quasi-Newton method.

11. The system (100) of any one of the preceding claims, wherein the trained model comprises a non-linear model, for example, a neural network.

12. The system (100) of any one of the preceding claims, wherein the trained model comprises a neural network, the neural network comprising multiple layers, the processor subsystem (140) being configured to iteratively adapt the set of parameters of the adapted model by iteratively adapting weights of only a subset of the multiple layers of the neural network.

13. A computer-implemented method (400) of processing a model trained based on a loss function, the model being configured to provide a model output given an input instance, the model being trained on a training dataset by iteratively optimizing an objective function, the objective function including respective losses according to the loss function for respective training instances of the training dataset, the method comprising:

    - accessing (410) the model and the training dataset on which the model has been trained;
    - receiving (420) a removal request message, the removal request message identifying one or more undesired training instances of the training dataset;
    - upon receiving the removal request message, making the model independent from the one or more undesired training instances by:

        - removing (430) the one or more undesired training instances from the training dataset to obtain a remainder dataset;
        - determining (440) an adapted model for the remainder dataset by:

            - initializing (442) a set of parameters of the adapted model based on the set of parameters of the trained model;
            - iteratively adapting (444) the set of parameters of the adapted model by optimizing the objective function with respect to the remainder dataset.

14. The method (400) of claim 13, further comprising obtaining a query instance and applying the adapted model to the query instance to obtain a model output independent from the one or more undesired training instances.

15. A computer-readable medium (500) comprising transitory or non-transitory data (510) representing instructions which, when executed by a processor system, cause the processor system to perform the computer-implemented method according to claim 13 or 14.

Fig. 1

210

220

RRM → REM

TM

PAR1 ... PARk — 230

231 232

250

TD — 240

TI1 — 241

...

TIi — 242

...

TIj — 243

TIn — 244

UTI — 245

MAD

ATM

PAR1' ... PARk'

261 262

260

II — 270

MAP — 280

MO — 290

Fig. 2

Fig. 3

400

410

420

430

440

442

444

Fig. 4

500    510

Fig. 5

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 10 225 277 B1 (SHINTRE SAURABH [US] ET AL) 5 March 2019 (2019-03-05)<br>* col.1 l.47-col.2 l.8, col.3 l.57-col.4 l.11, col.5 l.20-21, l.34-col.7 l.20, col.8 l.1-64;<br>figure 2 * | 1-15<br>1-15 | INV.<br>G06F21/57<br>G06F21/62<br>G16H50/20<br>G16H50/70<br>G16H10/20<br>G16H10/60 |
| X<br><br><br><br><br><br><br>Y | SHINTRE S ET AL: "Making Machine Learning Forget",<br>8 June 2019 (2019-06-08), ROBOCUP 2008: ROBOCUP 2008: ROBOT SOCCER WORLD CUP XII; [LECTURE NOTES IN COMPUTER SCIENCE; LECT.NOTES COMPUTER], SPRINGER INTERNATIONAL PUBLISHING, CHAM, PAGE(S) 72 - 83, XP047509806,<br>ISBN: 978-3-319-10403-4<br>[retrieved on 2019-06-08]<br>* the whole document * | 1-15<br><br><br><br><br><br><br>1-15 | G05B13/00<br>G06N3/08<br>A61B5/02 |
| X<br><br><br><br>Y | BOURTOULE L ET AL: "Machine Unlearning", ARXIV.ORG, CORNELL UNIVERSITY LIBRARY, 201 OLIN LIBRARY CORNELL UNIVERSITY ITHACA, NY 14853,<br>9 December 2019 (2019-12-09), XP081547745,<br>* the whole document * | 1-15<br><br><br><br>1-15 | TECHNICAL FIELDS SEARCHED (IPC)<br><br>G06F<br>G06N<br>G05B |
| Y | US 2018/101766 A1 (HE XI [US] ET AL) 12 April 2018 (2018-04-12)<br>* [0007] * | 1-15 | A61B<br>G16H |
| A | US 2019/139641 A1 (ITU LUCIAN MIHAI [RO] ET AL) 9 May 2019 (2019-05-09)<br>* [0004]-[0005] * | 3 | |

-/--

The present search report has been drawn up for all claims

| Place of search | Date of completion of the search | Examiner |
|---|---|---|
| Munich | 22 June 2020 | Werner, Andreas |

EPO FORM 1503 03.82 (P04C01)

2

Europäisches
Patentamt

European
Patent Office

Office européen
des brevets

**EUROPEAN SEARCH REPORT**

Application Number

EP 20 15 0536

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | KOH P W ET AL: "Understanding Black-box Predictions via Influence Functions", ARXIV.ORG, CORNELL UNIVERSITY LIBRARY, 201 OLIN LIBRARY CORNELL UNIVERSITY ITHACA, NY 14853, 14 March 2017 (2017-03-14), XP080756783, * the whole document * | 1-15 | |

TECHNICAL FIELDS
SEARCHED      (IPC)

The present search report has been drawn up for all claims

| Place of search | Date of completion of the search | Examiner |
|---|---|---|
| Munich | 22 June 2020 | Werner, Andreas |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**EP 3 848 836 A1**

## ANNEX TO THE EUROPEAN SEARCH REPORT
## ON EUROPEAN PATENT APPLICATION NO.

EP 20 15 0536

This annex lists the patent family members relating to the patent documents cited in the above-mentioned European search report.
The members are as contained in the European Patent Office EDP file on
The European Patent Office is in no way liable for these particulars which are merely given for the purpose of information.

22-06-2020

| Patent document cited in search report | | Publication date | Patent family member(s) | | Publication date |
|---|---|---|---|---|---|
| US 10225277 | B1 | 05-03-2019 | US | 10225277 B1 | 05-03-2019 |
| | | | US | 10397266 B1 | 27-08-2019 |
| US 2018101766 | A1 | 12-04-2018 | NONE | | |
| US 2019139641 | A1 | 09-05-2019 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **K. CHAUDHURI ; C. MONTELEONI.** Privacy-preserving logistic regression. *Advances in Neural Information Processing Systems,* 2009, 289-296
- **BARAK A. PEARLMUTTER.** Fast Exact Multiplication by the Hessian. *Neural computation,* 1994, vol. 6 (1), 147-160
- Variational Model Selection for Sparse Gaussian Process Regression. Technical report. University of Manchester, 2009
- **HENSMAN, FUSI ; LAWRENCE.** *Gaussian Processes for Big Data,* 2013, https://arxiv.org/abs/1309.6835
- **SALIMBENI ; DEISENROTH.** *Doubly Stochastic Variational Inference for Deep Gaussian Processes,* 2017, https://arxiv.org/abs/1705.08933
- **BARAK PEARLMUTTER.** *Fast exact multiplication by the Hessian", Neural Computation,* 1994, vol. 6 (1), 147-160
- **P. KOH ; P. LIANG.** Understanding black-box predictions via influence functions. *Proceedings of ICML,* 2017